# EUROPÄISCHE PATENTSCHRIFT

(11) **EP 1 299 523 B1**
(45) Veröffentlichungstag und Bekanntmachung des Hinweises auf die Patenterteilung: **24.12.2008**
(21) Anmeldenummer: 01965079.5
(22) Anmeldetag: 06.07.2001
(51) Int. Cl.: C12N 5/06

(54) **VERFAHREN ZUR MODIFIZIERUNG VON BIOLOGISCHEN ZELLEN**
METHOD FOR THE MODIFICATION OF BIOLOGICAL CELLS
PROCEDE POUR LA MODIFICATION DE CELLULES BIOLOGIQUES

(30) Priorität: 10.07.2000 DE 10033470; 30.10.2000 DE 10053783; 28.02.2001 DE 10109618
(43) Veröffentlichungstag der Anmeldung: 09.04.2003
(73) Patentinhaber: EPPENDORF AG, 22339 Hamburg (DE)
(72) Erfinder: ZIMMERMANN, Ulrich, 97295 Waldbrunn (DE); LUCAS, Kurt, 22359 Hamburg (DE); KÖPPEN, Barbara, 22523 Hamburg (DE)
(74) Vertreter: Hertz, Oliver
(86) Internationale Anmeldenummer: PCT/EP2001/007789
(87) Internationale Veröffentlichungsnummer: WO 2002/004603

(56) Entgegenhaltungen:
- SCOTT-TAYLOR ET AL.: "Human tumour and dendritic cell hybrids generated by electrofusion: potential for cancer vaccines" BIOCHIMICA ET BIOPHYSICA ACTA, Bd. 1500, 17. März 2000 (2000-03-17), Seiten 265-279, XP004276952
- CELLUZZI & FALO: "Cutting Edge: Physical interaction between dendritic cells and tumor cells result in an immunogen that induces protective and therapeutic tumor rejection" J. IMMUNOLOGY, Bd. 160, 1. April 1998 (1998-04-01), Seiten 3081-3085, XP002190766
- YANG ET AL.: "Immunotherapeutic potential of tumor antigen-pulsed and unpulsed dendritic cells generated from murine bone marrow" CELLULAR IMMUNOLOGY, Bd. 179, 1. Juli 1997 (1997-07-01), Seiten 84-95, XP001056634
- MACKENSEN ET AL: 'Phase I study in melanoma patients of a vaccine with peptide-pulsed dentritic cells generated in vitro from CD34+ hematopoietic progenitor cells' INT. J. CANCER Bd. 86, 2000, Seiten 385 - 392, XP001056637

## Beschreibung

Die Erfindung betrifft Verfahren zur Handhabung und/oder Behandlung von biologischen und/oder synthetischen Zellen, insbesondere zur Wechselwirkung von Zellen oder Zellbestandteilen, die Antigene darstellen oder tragen (z. B. Tumorzellen), und Zellen, die Antigene präsentieren (wie z. B. Mastzellen oder dendritische Zellen), Verfahren zur Erzeugung von Verbunden aus Antigene präsentierenden Zellen und erkrankten Zellen, z. B. Tumorzellen, oder deren Zellbestandteilen (z. B. Membranteilen), Verfahren zur Modifizierung von Antigene präsentierenden Zellen, z. B. dendritischen Zellen, mit erkrankten Zellen, z. B. Tumorzellen, oder deren Zellbestandteilen (z. B. Membranteilen), Verfahren zur passiven Immunisierung oder Impfung von Organismen gegen Erkrankungen (z. B. virale oder bakterielle Infektionen, Tumorerkrankungen oder dergleichen), Verfahren zur Behandlung von Infektions- oder Tumorerkrankungen und/oder Verfahren zur Herstellung von Zusammensetzungen zur passiven Immunisierung oder Impfung von Organismen gegen Infektions- oder Tumorerkrankungen. Vorrichtungen zur Umsetzung der Verfahren, Verwendungen von Vorrichtungen zur dielektrophoretischen Manipulierung von Zellen zur Modifizierung von Antigene präsentierenden Zellen, insbesondere dendritischen Zellen oder Mastzellen, und Anwendungen der genannten Verfahren werden ebenfalls offenbart.

Es gibt Versuche, zur Tumorbehandlung einen Organismus einer Anregung des Immunsystems zu unterziehen, indem körpereigene oder fremde dendritische Zellen mit Antigenen modifiziert und dem Organismus zugeführt werden. Die dendritischen Zellen wirken als immunoaktive Zellen, die in Abhängigkeit von den jeweiligen Antigenen immunostimulierende Eigenschaften besitzen. Die Bildung und Eigenschaften von dendritischen Zellen werden bspw. von K. Shortman et al. in "Stem Cells", Band 15, 1997, Seite 409 ff. beschrieben. Die Modifizierung der dendritischen Zellen mit Antigenen bedeutet, dass die Antigene in die Oberfläche der dendritischen Zelle eingebaut werden. Als Antigene werden bspw. bestimmte Modellpeptide (siehe P. Paglia et al. in "Minerva Biotecnologica", Band 11, 1999, Seite 261 ff.) oder von den jeweiligen Tumorzellen gebildete Antigene verwendet. Zur Beladung der dendritischen Zellen mit Antigenen von Tumorzellen sind die folgenden Verfahren bekannt.

Bei dem bspw. von T. H. Scott-Taylor et al. in "Biochimica et Biophysica Acta", Band 1500, 2000, Seite 265 ff. beschriebenen Verfahren werden Tumorzellen mittels Elektrofusion mit dendritischen Zellen verschmolzen, die mit den Tumorzellen insbesondere die Tumor-Antigene aufnehmen und dadurch eine Anregung des Immunsystems auslösen können. Dieses Verfahren besitzt den Nachteil, dass die Elektrofusion ein komplexer Vorgang ist, der eine anwendungsspezifische Optimierung der Fusionsbedingungen erfordert. Die Elektrofusion von dendritischen.Zellen mit Tumorzellen hat ferner den Nachteil, dass die in der Literatur beschriebenen Ausbeuten sehr gering sind und dass, bedingt durch die zufällige Kombination der Gene der beiden Fusionspartner, Fusionsprodukte mit unterschiedlichen Eigenschaften entstehen, die nach Rückgabe in den Patienten zu unterschiedlichen Immunreaktionen führen können. Um nicht den zu behandelnden Organismus noch mit Tumorzellen zu belasten, müssen die Tumorzellen vor der Elektrofusion, bspw. mittels radioaktiver Bestrahlung, abgetötet werden. Es bleibt jedoch bei den üblichen Bestrahlungsmethoden ein Restrisiko, dass Tumorzellen überleben und zu Metastasen führen. Damit besitzen Immunisierungsverfahren, die auf der Elektrofusion ganzer Zellen beruhen, nur eine eingeschränkte Zuverlässigkeit, da gewährleistet sein muss, dass die Tumorzellen alle abgetötet sind sowie alle Krankheitserreger, die sich in den Tumorzellen befinden, wie z. B. Viren, ausgeschaltet sind.

Aus der Publikation von K. Shimizu et al. in "Proc. Natl. Acad. Sci. USA", Band 96, 1999, Seite 2268, ist die Verwendung von Tumorzell-Lysaten zur Modifizierung der dendritischen Zellen bekannt. Die Lysate sind Tumorzellen, die durch einen Einfrier- und Auftauvorgang zerstört worden sind. Die Interaktion der dendritischen Zellen mit den Lysaten wird durch eine Langzeitinkubation über rund 20 Stunden induziert. Die Verwendung der Lysate besitzt neben dem großen Zeitaufwand auch den Nachteil einer eingeschränkten Zuverlässigkeit. Wie bei der Elektrofusion ist eine radioaktive Bestrahlung vorgesehen, um die Gefahr einer zusätzlichen Metastasierung zu vermindern.

Yang et al. beschreiben in Cell. Immunology, 179, 84-95 (1997) ein sehr ähnliches Verfahren mit Tumorzellextrakten, die ebenfalls durch Einfrieren und Auftauen hergestellt wurden. Die Verwendung dieser Extrakte ist durch die Anwesenheit von Tumorzell-DNA mit den gleichen Nachteilen wie bei dem von Shimizu et al. beschriebenen Verfahren verbunden.

Die Erzeugung einer Assoziation aus dendritischen Zellen und Tumorzellen wird auch von C. M. Celluzzi et al. in "The Journal of Immunology", Band 160, 1998, Seite 3081 ff. beschrieben. Die Assoziation erfolgt entweder durch eine elektrisch stimulierte Zellfusion oder durch eine physikalische Wechselwirkung bei einer Langzeitinkubation, die zu einer sogenannten Scheinfusion (mock fusion) führt. Auch bei diesem Verfahren treten die genannten Probleme auf. Die zur Auslösung der Immunreaktion verwendete Zellassoziation trägt mit dem Zellkern der Tumorzelle ein erhebliches Metastasierungsrisiko in sich. Außerdem erfordert die Bildung von Scheinfusionen eine zeitaufwendige Koinkubation mit einer Dauer von mehr als zehn Stunden.

Aus der Publikation von N.-S. Yang et al. ("Proc. Natl. Acad. Sci. USA" Bd. 87, 1990, S. 9568 ff.) ist eine physikalische Methode zur Einbringung von DNA-Molekülen mit beschleunigten Festpartikeln (sogenannte Mikroprojektile) in Zellen bekannt. Die Mikroprojektile sind beschichtete Gold- oder Wolframpartikel mit einer Größe von 1 µm. Die DNA wird auf der Partikeloberfläche gebunden. Das Einschießen mit Geschwindigkeiten im Bereich von 500 m·s⁻¹ erfolgt unter Verwendung von Druckquellen oder elektrischen Feldern. Dieses Verfahren zur DNA-Transfektion wird auch als biolistischer Partikeleinschuß bezeichnet.

Die Aufgabe der Erfindung ist es, neue Verfahren zur Behandlung biologischer Zellen anzugeben, bei dem Zellen miteinander zur Wechselwirkung gebracht werden. Es soll insbesondere ein verbessertes Verfahren zur Modifizierung dendritischer Zellen geschaffen werden, mit dem die Nachteile herkömmlicher Techniken überwunden werden und das sich durch einen schnellen Verfahrensablauf und den Ausschluss eines Metastasierungsrisikos auszeichnet. Das erfindungsgemäße Verfahren soll auch in Bezug auf die Reproduzierbarkeit der Einstellung von Wechselwirkungsbedingungen verbessert sein. Es soll ferner ein neues, erweitertes Verfahren zur Modifizierung von Zellen in Suspensionen geschaffen werden.

Eine erste Grundidee der Erfindung ist es, biologische und/oder synthetische Zellen, Zellbestandteile oder Makromoleküle miteinander zu Wechselwirkungen zu veranlassen, indem die jeweiligen Teilchen miteinander in Kontakt gebracht werden, wobei sich die zur Wechselwirkung gebrachten Zellen, Zellbestandteile oder Makromoleküle in einem suspendierten Zustand in einer Suspension befinden. Es erfolgt eine Modifizierung von dendritischen Zellen oder anderen Antigene präsentierenden Zellen mit erkrankten Zellen im suspendierten Zustand. Antigene präsentierende Zellen und erkrankte Zellen oder Zellbestandteile von erkrankten Zellen (z. B. Tumorzellen oder Tumorzellbestandteile, Bakterien, Viren/Virenhüllen, Stammzellen, Knochenmarkzellen oder Epithelzellen) werden in einer Suspension äußeren Kräften derart ausgesetzt, dass die verschiedenen Zelltypen oder Zellbestandteile sich gegenseitig berühren. Der Erfinder hat festgestellt, dass eine Berührung unter Einwirkung äußerer Kräfte ausreicht, um ausschließlich Membrankomponenten mit den Antigenen von den erkrankten Zellen oder Zellbestandteilen auf die Antigene präsentierenden Zellen zu übertragen. Zellkerne und andere Bestandteile der erkrankten Zellen bleiben in der Suspension. Die modifizierte Antigene präsentierenden Zellen werden von den noch freien erkrankten Zellen oder Zellbestandteilen in der Suspension getrennt und für die jeweiligen Anwendung, insbesondere die Anregung des Immunsystems eines Organismus, bereitgestellt. Gemäß einem bevorzugten Aspekt der Erfindung wird das Verfahren zur Erzeugung immunologisch aktiver Zellen, insbesondere immunologisch aktiver dendritischer Zellen, verwendet. Gegenstand der vorliegenden Erfindung ist insbesondere die Modifizierung von suspendierten Antigene präsentierenden Zellen, wie T-Zellen, B-Zellen oder Mastzellen.

Unter Wechselwirkung der Zellen oder Zellbestandteile wird allgemein jede Art von mechanischer oder stofflicher oder anderweitiger Wechselwirkung, insbesondere Wechselwirkungen, bei denen eine Substanzübertragung oder eine Übertragung von Zellbestandteilen z. B. auf eine dendritische Zelle erfolgen, betrachtet. Die Zellen werden durch Bildung chemischer Bindungen oder physikalischer Assoziationen gekoppelt, so dass sich die Zellmembranen berühren. Wenn sich die Zellmembranen berühren, kommt es zur gegenseitigen Wechselwirkung. Es werden bspw. Membranstücke des einen Zelltyps auf den anderen Zelltyp übertragen.

Eine weitere Grundidee der Erfindung ist es, biologische und/oder synthetische Zellen, Zellbestandteile oder Makromoleküle miteinander zu Wechselwirkungen zu veranlassen, indem die jeweiligen Teilchen miteinander in Kontakt gebracht werden, wobei sich mindestens eine Gruppe der zur Wechselwirkung gebrachten Zellen, Zellbestandteile oder Makromoleküle in einem adhärenten Zustand auf einem Festphasensubstrat befindet. Das Festphasensubstrat wird durch ein planares Substrat, z. B. Substrat mit mindestens einer Glas-, Kunststoff- oder Membranoberfläche, oder ein partikelförmiges Substrat gebildet. Das Festphasensubstrat mit den adhärenten Zellen wird in eine Flüssigkeit eingebracht, in der die jeweils andere Gruppe von Zellen, Zellbestandteilen oder Makromolekülen suspendiert ist. Die suspendierten Teilchen werden in der Suspension äußeren Kräften derart ausgesetzt, dass die verschiedenen Zelltypen oder Zellbestandteile sich gegenseitig berühren. Die Erfinder haben festgestellt, dass eine Berührung unter Einwirkung äußerer Kräfte ausreicht, um ausschließlich Membrankomponenten mit den Antigenen von den erkrankten Zellen oder Zellbestandteilen auf die Antigene präsentierenden, z. B. dendritischen, Zellen zu übertragen. Zellkerne und andere Bestandteile der erkrankten Zellen oder Zellbestandteile bleiben in der Suspension.

Gemäß einer bevorzugten Ausführungsform der Erfindung sind die Antigene präsentierenden Zellen auf dem Festphasensubstrat gebunden, das in der Suspensionsflüssigkeit angeordnet ist. Die erkrankten Zellen oder Zellbestandteile, von denen Antigene auf die adsorbierten Zellen übertragen werden sollen, werden in die Suspension gegeben. Nach der gegenseitigen Wechselwirkung werden die modifizierten adsorbierten Zellen von den noch freien erkrankten Zellen oder Zellbestandteilen in der Suspension getrennt und für die jeweiligen Anwendung, insbesondere die Anregung des Immunsystems eines Organismus, bereitgestellt. Die Trennung erfolgt vorzugsweise durch Entfernung der erkrankten Zellen oder Zellbestandteile aus der Suspension oder Überführung des Festphasensubstrats in eine andere Umgebung. Anschließend werden die modifizierten Zellen vom Festphasensubstrat abgelöst. Dies erfolgt beispielsweise durch einen enzymatischen Prozess. Gemäß einem bevorzugten Aspekt der Erfindung wird das Verfahren zur Erzeugung immunologisch aktiver dendritischer Zellen verwendet.

Gemäß einer abgewandelten Ausführungsform der Erfindung werden die Antigene präsentierenden Zellen mit erkrankten Zellen im adsorbierten Zustand in Kontakt gebracht, so dass Antigene der erkrankten Zellen auf die Antigene präsentierenden Zellen übertragen werden. Die Übertragung der Antigene erfolgt wiederum, indem Membranbestandteile der erkrankten Zellen an den Antigene präsentierenden Zellen anhaften oder in deren Membranen eingebaut oder aufgenommen werden.

Ein entscheidender Vorteil der Erfindung besteht darin, dass die Modifizierung der Antigene präsentierenden Zellen innerhalb kurzer Zeiten wie bei der herkömmlichen Elektrofusion durchgeführt werden kann, wobei jedoch gegenüber den herkömmlichen Fusions- oder Scheinfusionstechniken vorteilhafterweise nur Zellbestandteile mit den gewünschten Antigenen, nicht jedoch ganze Tumorzellen an die dendritischen Zellen angekoppelt werden. Damit wird erstmalig das Metastasierungsrisiko bei Anwendung der dendritischen Zellen eliminiert.

Die Bindung der Gruppe der Antigene übertragenden Zellen oder der Gruppe der Antigene präsentierenden Zellen an ein Festphasensubstrat besitzt den zusätzlichen Vorteil, dass die Parameter der gegenseitigen Wechselwirkung, insbesondere ausgeübte Kräfte, Wechselwirkungsdauer, Temperatur, beteiligte Substanzen, mit hoher Genauigkeit, Reinheit und Reproduzierbarkeit eingestellt werden können.

Als Verfahren zur Kontaktierung und Aneinanderkopplung der Zellen durch Ausübung äußerer Kräfte sind alle an sich bekannten Verfahren zur Manipulierung von Zellen oder Partikeln verwendbar, wie z. B. die Dielektrophorese, die Sedimentation, die Zentrifugation, der hypo-osmolare Schock, die Ausübung von Strömungskräften (z. B. Vermischung und Schütteln), Filtertechniken, die optische Manipulierung mit Laserpinzetten oder mechanische Manipulierung, wie z. B. Partikeleinschluss, und dergleichen. Die äußeren Kräfte werden so stark eingestellt, dass die Zellen auch im aneinanderhaftenden Zustand bleiben, wenn die Kraftwirkung abgeschaltet oder beendet wird.

Zur Modifizierung von Antigene präsentierenden Zellen mit erkrankten Zellen oder Zellbestandteilen, wie z. B. Membranvesikeln, die vorteilhafterweise leicht aus erkrankten Zellen, z. B. Tumorzellen, oder Zellbestandteilen oder Partikeln, z. B. Virushüllen, gewonnen werden können, werden beide Zelltypen in einen Wechselwirkungsbereich gebracht und dort äußeren Kräften zur gegenseitigen Kontaktierung ausgesetzt. Die Kräfte zur Kontaktierung werden anwendungsabhängig so lange ausgeübt, bis sich die gewünschte chemische oder physikalische Bindung ausgebildet hat. Dies wird ggf. durch Beobachtung oder Vermessung der Zellen oder des Zellverbundes festgestellt.

Es können gleichzeitig einzelne oder mehrere Zellen beider Zelltypen zur Wechselwirkung gebracht werden.

Nach der Kontaktierung ist es, in Abhängigkeit von den spezifischen Eigenschaften der erkrankten Zellen, unter Umständen vorteilhaft, den Einbau der Antigene in den zu modifizierenden Zellen durch einen Feldpuls, der zum reversiblen dielektrischen Durchbruch der Membran führt, zu verstärken (z. B. feld-induzierte Endozytose). Es wird, z. B. mit einer Elektrodeneinrichtung, wie sie an sich zur Elektroporation von Zellen bekannt ist, ein Durchbruchpuls in hypo- oder iso-osmolarer Lösung induziert. Dies hat den Vorteil, dass Membranbestandteile besonders schnell von den zu modifizierenden Zellen aufgenommen werden.

Gemäß einer weiteren Ausführungsform der Erfindung werden die Antigene präsentierenden Zellen mit Membranbestandteilen von erkrankten Zellen in Kontakt gebracht, um die Antigene von diesen zu übernehmen. Die Übertragung der Antigene erfolgt, indem Membranbestandteile der erkrankten Zellen an den Antigene repräsentierenden Zellen anhaften oder an deren Membranen eingebaut oder aufgenommen werden. Hierzu werden die Membranbestandteile zunächst von den Kernen und dem Zytoplasma der erkrankten Zellen abgetrennt und dann mit den Antigene präsentierenden Zellen zur Wechselwirkung gebracht. Als Membranbestandteile werden vorzugsweise Membranbruchstücke, die sich spontan zu Membranvesikeln formieren, verwendet.

Gemäß einem weiteren Gesichtspunkt der Erfindung wird ein Verfahren zur passiven Immunisierung oder Impfung von Organismen gegen Erkrankungen, insbesondere Tumorerkrankungen, bereitgestellt. Zur Umsetzung des Verfahrens werden allogene, z. B. dendritische Zellen, d. h. Zellen von einem anderen (gesunden) Menschen, sowie z. B. Tumorzellen des Patienten entnommen, gegen den die behandelten Zellen immunologisch aktiviert werden sollen. Die entnommenen Zellen werden einem der genannten Verfahren zur gegenseitigen Wechselwirkung unterzogen, so dass die dendritischen Zellen Tumor-Antigene aufnehmen. Die behandelten dendritischen Zellen werden anschließend dem Probanden (Patienten) rückinjiziert. Als Tumorzellen können auch bereits bestrahlte (abgetötete) Tumorzellen verwendet werden. Eine erfindungsgemäße passive Immunisierung kann analog auch gegen bakterielle oder virale Infektionen erfolgen.

Gegenstand der Erfindung ist auch ein zellulärer Impfstoff, der Zellen enthält, die mit Antigentragenden Membrankomponenten von erkrankten Zellen modifiziert sind.

Ebenfalls offenbart sind Vorrichtungen zur Manipulierung biologischer Zellen. Eine solche Vorrichtung enthält eine Einrichtung zur Lagerung und Zuführung von Zellen oder Zellbestandteilen in einen Wechselwirkungsbereich (z. B. Flüssigkeitsbehälter), eine Manipulierungseinrichtung im Wechselwirkungsbereich, z. B. ein Mikroelektrodensystem zur dielektrophoretischen Manipulierung von Zellen, ggf. eine Mess- und Beobachtungseinrichtung zur Erfassung des Ergebnisses der Zellbehandlung, und eine Extraktionseinrichtung. Zur oben genannten Modifizierung von Festphasen-adsorbierten Zellen enthält der Wechselwirkungsbereich ein Festphasensubstrat für Zellen, die zu modifizieren sind oder die der Modifizierung anderer Zellen dienen.

Die Erfindung wird vorzugsweise zur passiven Immunisierung oder Impfung gegen Tumorwachstum angewendet.

Weitere Einzelheiten und Vorteile der Erfindung werden aus der folgenden Beschreibung von Ausführungsbeispielen unter Bezug auf die beigefügten Zeichnungen ersichtlich. Es zeigen:
- Figur 1: eine schematische Illustration der erfindungsgemäßen Modifizierung dendritischer Zellen, und
- Figuren 2 bis 4: graphische Darstellungen experimenteller Ergebnisse, die mit erfindungsgemäß modifizierten Zellen erzielt wurden,
- Figur 5: eine schematische Illustration der erfindungsgemäßen Festphasen-Modifizierung dendritischer Zellen, und
- Figur 6: schematische Schnittansichten erfindungsgemäß verwendbarer Vorrichtungen.

Das Grundprinzip der Erfindung, nämlich der Einbau von Antigenen in Antigene präsentierende Zellen durch deren Kontaktierung mit erkrankten.Zellen oder Zellbestandteilen unter Wirkung äußerer Kräfte, kann jeweils mit dem Ziel einer Immuntherapie mit den verschiedensten Typen erkrankter Zellen realisiert werden. Als Antigene präsentierende Zellen können erfindungsgemäß insbesondere dendritische Zellen, T-Zellen, B-Zellen oder Mastzellen verwendet werden. Die Erfindung wird im folgenden ohne Beschränkung am Beispiel der Modifizierung dendritischer Zellen beschrieben. Als "Antigen" werden hier alle biologischen Moleküle verstanden, auf die ein Organismus mit einer Immunantwort reagiert. Die Antigene können natürlichen oder artifiziellen Ursprungs sein. Sie können von Zellen, Zellbestandteilen, synthetischen Teilchen (wie z. B. Membranvesikeln) oder frei aus der Suspension auf die zu modifizierenden Zellen übertragen werden. Neben Tumorzellen können bspw. auch Bakterien, Viren/Virenhüllen, Stammzellen, Knochenmarkzellen oder Epithelzellen verwendet werden, um Antigene oder Membranbestandteile dieser Zellen auf die dendritischen Zellen zu übertragen. Im Folgenden wird die Erfindung ohne Einschränkung in Bezug auf die Modifizierung festphasenadsorbierter, dendritischer Zellen mit Tumorzellen erläutert.

Des Weiteren ist die Erfindung durch die Ankopplung ganzer erkrankter Zellen, die ggf. vorher abgetötet wurden, oder von deren Membranbestandteilen an die dendritischen Zellen umsetzbar. Die Membranbestandteile werden entweder aus den erkrankten Zellen zuerst gewonnen und dann mit den dendritischen Zellen zur Wechselwirkung gebracht oder von den erkrankten Zellen während der Kontaktierung mit den dendritischen Zellen auf diese übertragen. Die Wechselwirkung der dendritischen Zellen mit den erkrankten Zellen besitzt den Vorteil eines vereinfachten Verfahrensablaufes, da der Schritt der gesonderten Bereitstellung der Membranbestandteile entfällt. Es müssen jedoch besondere Maßnahmen zum Metastasierungs- oder Infektionsschutz getroffen werden, falls die erkrankten Zellen komplett an die dendritischen Zellen angekoppelt werden. Werden die Membranbestandteile gesondert präpariert, besitzt dies den Vorteil, dass die modifizierten dendritischen Zellen in ihrer Größe und in ihren funktionellen Eigenschaften kaum verändert sind. Die modifizierten Zellen bewegen sich auf dem Weg zum Lymphknoten und verhalten sich dort wie unmodifizierte Zellen. Damit wird die immunstimulierende Funktion der dendritischen Zellen im Vergleich zur Zell-ZellFusion verbessert. Im Folgenden wird ohne Einschränkung vorrangig auf die Wechselwirkung dendritischer Zellen mit Membranbestandteilen, die gesondert präpariert wurden, Bezug genommen.

Ein wesentliches Merkmal der Erfindung besteht darin, dass die frei suspendierten oder an einer Festphase adsorbierten dendritischen Zellen einerseits und die erkrankten Zellen oder deren Zellbestandteile andererseits in einer gemeinsamen Suspension äußeren Kräften ausgesetzt werden. Dies ermöglicht eine Kurzzeitkontaktierung, die je nach Art der äußeren Kräfte im Bereich von Mikrosekunden oder Millisekunden, wenigen Minuten oder bis zu ein bis zwei Stunden liegen kann. Damit wird gegenüber herkömmlichen Verfahren eine erhebliche Reduzierung der Behandlungsdauer erzielt. Der Anteil der dendritischen Zellen, die die Behandlung ohne Funktionseinbuße überleben, wird damit erhöht. Die Wirksamkeit des erfindungsgemäßen Impfstoffs auf der Grundlage modifizierter dendritischer Zellen wird erhöht. Im Folgenden wird ohne Einschränkung auf die Ausübung von Strömungskräften in der gemeinsamen Suspension, z. B. durch Bewegung des die Suspension enthaltenden Gefäßes oder anderweitiges Vermischen, und/oder elektrischen Kräften Bezug genommen. Die äußeren Kräfte können analog durch die dielektrophoretische Kräfte, Zentrifugationskräfte, optische Kräfte und/oder die weiteren, oben genannten Kräfte ausgeübt werden. Dabei sind die entsprechenden, an sich bekannten Techniken zur Handhabung von Zellen oder Zellbestandteilen einsetzbar. Vorteilhafterweise können große Zellzahlen (10⁶ bis 10⁸ oder mehr) behandelt werden.

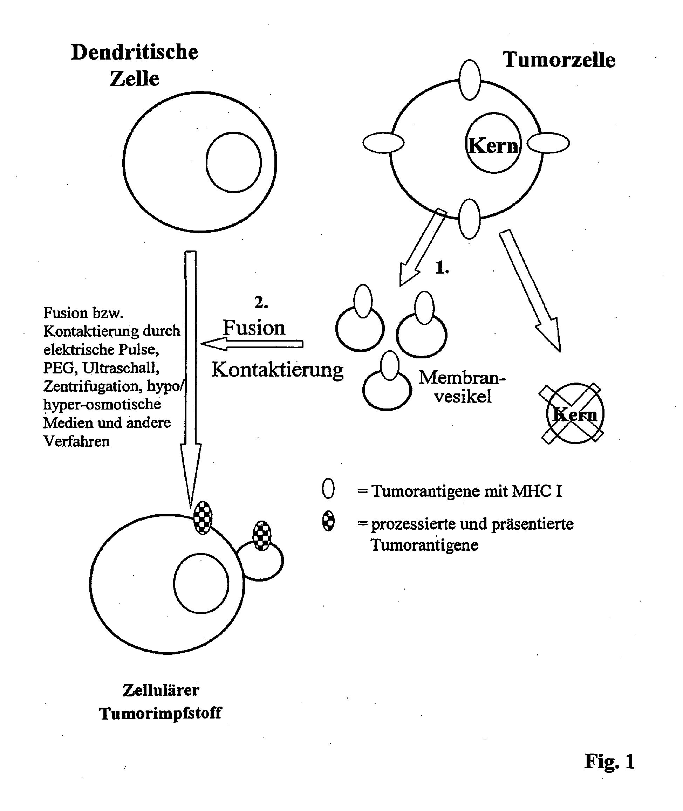

Figur 1 zeigt zur Illustration des erfindungsgemäßen Verfahrens zur Zellmodifizierung in Suspensionen schematisch den Präparationsschritt 1. zur Herstellung von Membranvesikeln aus Tumorzellen und den Kontaktierungsschritt 2. zur Modifizierung der dendritischen Zellen. Im Folgenden wird zunächst der Präparationsschritt an einem Verfahrensbeispiel und anschließend der Kontaktierungsschritt an verschiedenen Verfahrensbeispielen erläutert.

### 1. Präparationsschritt (Suspensions- oder Festphasenmodifizierung)

Beim Präparationsschritt werden Membranvesikel aus Tumorzellen durch ein an sich bekanntes Homogenisierungsverfahren mit anschließender Entfernung der Zellkerne hergestellt. Die Verfahrensweise wird bspw. von J. M. Graham et al. in "Molekularbiologische Membrananalyse", Spektrum Akademischer Verlag GmbH, 1998, erläutert.

Die Tumorzellen werden mit einem Glas-Potter homogenisiert. Es werden bspw. mit einem üblichen Labor-Potter (Spaltbreite z. B. 150 µm) 5-ml-Proben mehrfach (z. B. 15-fach) homogenisiert. Anschließend wird die homogenisierte Suspension zentrifugiert. Im Ergebnis der Zentrifugation befinden sich die schweren Zellbestandteile, insbesondere die Kerne und Organellen, im Pellet. Die Zellbestandteile geringerer Dichte (Membranbestandteile) befinden sich im Überstand. Es erfolgt bspw. eine Zentrifugation bei 3500 rpm (2000 · g) mit einer Dauer von 15 Minuten. Nach der Zentrifugation wird der Überstand vom Pellet getrennt. Aus den Membranbestandteilen bilden sich Membranvesikeln, d. h. geschlossene, lediglich mit der Suspensionsflüssigkeit gefüllte Membranhüllen in Kugelform. Die Membranvesikeln besitzen charakteristische Durchmesser von weniger als 1 µm. In den Membranen der Membranvesikeln sind die Tumorantigene mit dem immunstimulierenden MHCI-Komplex enthalten. Die Vesikelsuspensionen werden z. B. als 4-ml-Proben im Kühlschrank gelagert. Die ursprüngliche Osmolalität der Vesikelsuspensionen wird zur Vermeidung einer Verletzung der Membranvesikeln durch osmotischen Druck von zunächst 20 mOsm auf eine höhere Osmolalität (z. B. 80 mOsm) eingestellt. Dies erfolgt bspw. mit 10-facher PBS (80 µl 10-fach PBS + 4 ml Vesikelsuspension).

Im Ergebnis des Präparationsschrittes 1. ist der Zellkern von den Membranvesikeln getrennt, wie dies in Figur 1 schematisch illustriert ist. Die erfindungsgemäße Modifizierung der dendritischen Zellen erfolgt vorzugsweise lediglich mit den Membranvesikeln, die die Tumorantigene tragen.

Für Analyse- oder Testzwecke (experimentelle Ergebnisse siehe unten) kann eine Einfärbung der Membranproteine vorgesehen sein. Zur Einfärbung wird zunächst die Tumorzellsuspension mit dem Markierungsfarbstoff (z. B. FITC) versetzt. Anschließend erfolgt eine Entfernung des ungebundenen Farbstoffes aus der Suspension. Die Zellsuspension (1.2 ml, PBS, 1 · 10⁷/ml) wird mit 50 ... 150 µM FITC (36 µl, Stammlösung 5 mM in DMF) versetzt. Die Färbung dauert bei 37°C rund 5 ... 15 min. Zur Entfernung des ungebundenen Farbstoffes erfolgen mehrere Waschschritte mit einem den restlichen Farbstoff bindenden Protein (z. B. mit PBS-BSA, 20°C, 1 % BSA), jeweils kombiniert mit Zentrifugationsschritten. Anschließend erfolgt ein Waschen in einer Pufferlösung, eine Zentrifugation und die Bereitstellung des Pellets mit den Tumorzellen in einem PMSF-Puffer, der hypo-osmolar ist, so dass die Tumorzellen anschwellen. Als PMSF-Puffer ist die folgende Zusammensetzung vorgesehen: 10 mM Tris, 0.5 mM Proteasen-Inhibitor PMSF (Phenylmethylsulfonylfluorid), pH 7.2.

### 2. Kontaktierungsschritt (Suspensions-Modifizierung)

### Osmotisch-induzierter Einbau der Vesikeln in dendritische Zellen

Zur Kontaktierung wird zunächst ein Ansatz der dendritischen Zellen als Zellsuspension oder als Zellprobe (ohne Suspensionsflüssigkeit) bereitgestellt. Der Ansatz wird dann mit der Vesikelsuspension in eine gemeinsame Suspension überführt, in der die Kontaktierung der Membranvesikeln mit den dendritischen Zellen unter Wirkung äußerer Kräfte erfolgt. Die Suspension dendritischer Zellen kann isoton oder hypoton gebildet sein. Die hypotone Suspension wird bevorzugt, da in dieser die dendritischen Zellen angeschwollen sind. Die unten erläuterten experimentellen Ergebnisse zeigen, dass die angeschwollenen dendritischen Zellen mit größerer Effektivität mit den Membranvesikeln modifizierbar sind. Zur Herstellung der isotonen Suspension wird z. B. eine Suspension von 10⁶ dendritischen Zellen/ml (2 ml) zunächst abzentrifugiert und dann in 5 ml PBS (280 mOsm) gewaschen und dann in 100 µl PBS (280 mOsm) aufgenommen. Zur Herstellung der hypotonen Suspension werden entsprechend 2 ml der Ausgangssuspension abzentrifugiert und dann in 5 ml PBS, verdünnt mit H₂O (80 mOsm), gewaschen und dann in 100 µl hypo-osmolarem PBS (80 mOsm) aufgenommen.

Die isotone oder hypotone Suspension wird dann mit der Vesikelsuspension (2 ml, 80 mOsm) zusammengeführt, um die erfindungsgemäße Modifizierung der dendritischen Zellen mit den Membranvesikeln zu bewirken. Hierzu erfolgt zunächst eine Inkubation bei 37°C (10 min). Anschließend erfolgt eine Einstellung der Suspension auf einen iso-osmolaren Zustand (z. B. mit 147 µl 10-fach PBS auf 280 mOsm). Dadurch werden die dendritischen Zellen verkleinert, es entsteht eine unregelmäßig gekrümmte Membranoberfläche, deren Gestalt den Einbau der Membranvesikel in die Membran, Endozytosevorgänge und ein äußeres Adherieren fördert. Es folgt eine Inkubation bei 37°C für rund 1.5 ... 2 Stunden. Während dieser Inkubation erfolgt die Modifizierung der dendritischen Zellen. Die äußeren Kräfte werden in Form von Strömungskräften ausgeübt. Anschließend erfolgt eine Waschung mit PBS (280 mOsm), um die nicht-angekoppelten (noch freien) Membranvesikel zu entfernen.

Die am Ende vorliegende Suspension enthält modifizierte dendritische Zellen als zellulären Tumorimpfstoff (siehe Figur 1, links unten), der die Antigene der Tumorzelle in der Membran direkt oder in angehefteten Membranbestandteilen enthält.

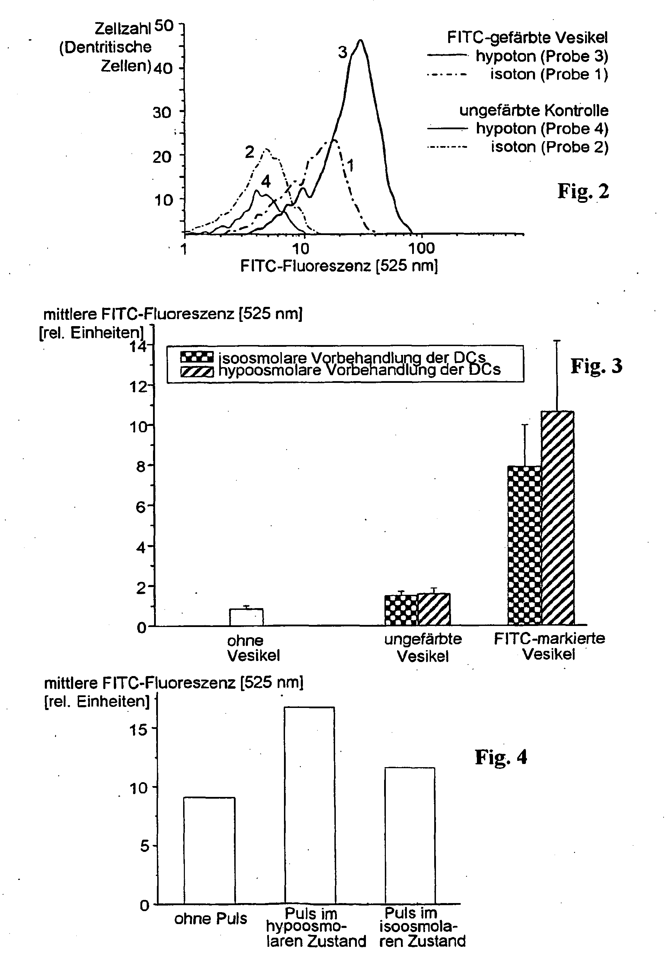

Figur 2 illustriert die Fluoreszenzanalyse von gefärbten Zellproben im Vergleich mit ungefärbten Kontrollproben mittels FACS-Analyse. Bei den Proben 1 und 3 wurden Suspensionen mit gefärbten Vesikeln einen isotonen (Probe 1) oder hypotonen (Probe 3) Ansatz dendritischer Zellen zugeführt. Bei den Kontrollproben 2 und 4 erfolgte entsprechend eine Zufuhr ungefärbter Vesikel. Es zeigt sich, dass eine stark erhöhte Intensität der FITC-Fluoreszenz bei 525 nm der dendritischen Zellen nach der Inkubation mit den FITC-markierten Membranvesikeln von Tumorzellen (H7-Zellen). Die Vorbehandlung der dendritischen Zellen mit hypotonem Medium (Probe 3) bewirkt eine erheblich höhere Inkorporation von Membranvesikeln als die isotone Vorbehandlung (Probe 1). Die Kurven der Kontrollproben 2 und 4 zeigen die erheblich schwächere Autofluoreszenz der dendritischen Zellen nach Fusion mit ungefärbten Vesikeln.

Eine quantitative Auswertung der Fluoreszenzanalyse wird in Figur 3 illustriert. Die FITC-Fluoreszenz wird als Maß für den Einbau der gefärbten Vesikel in die dendritischen Zellen verwendet. Die mit ungefärbten Vesikeln behandelten dendritische Zellen zeigen eine geringfügige Erhöhung der Autofluoreszenz. Die gefärbten Vesikeln ergeben deutlich erhöhte Fluoreszenzintensitäten, wobei die hypo-osmolare Vorbehandlung der dendritischen Zellen eine stärkere Fluoreszenz ergibt, die den effektiveren Einbau der gefärbten Vesikeln in die dendritischen Zellen bestätigt.

### Elektrisch-induzierter Einbau der Vesikeln in dendritische Zellen

Beim elektrisch-induzierten Einbau der Vesikeln werden zunächst eine isotone oder eine hypotone Suspension dendritischer Zellen wie oben beschrieben mit einer Vesikelsuspension zusammengeführt und für kurze Zeit (z. B. 5 min)bei Raumtemperatur inkubiert. Jeweils 800 µl der Suspension aus dendritischen Zellen und Vesikeln werden einem elektrischen Feldpuls ausgesetzt, der die äußeren Kräfte zur Modifizierung der dendritischen Zellen bildet. Die Parameter des Feldpulses betragen bspw. 1 kV/cm, Dauer: 20 µs. Zehn Minuten nach Pulsapplikation wurde die Osmolarität der hypotonen Suspension durch Zugabe von 10 x PBS auf 280 mOsm eingestellt. Nach der Ausübung der Feldpulse erfolgt eine Inkubation bei Raumtemperatur und anschließend zur Regenerierung der Zellen eine Inkubation bei 37°C für eine Stunde. Schließlich wurden die Proben mit PBS (280 mOsm) gewaschen, um die nicht fusionierten, freien Vesikeln zu entfernen.

Die FACS-Analyse der modifizierten dendritischen Zellen ist in Figur 4 illustriert. In Figur 4 sind die mittleren FITC-Fluoreszenzintensitäten bei 525 nm für die verschiedenen Proben illustriert. Die linke Säule zeigt die Fluoreszenz ohne Ausübung eines Feldpulses. Diese Probe entspricht somit dem oben erläuterten Verfahren der osmotisch-induzierten Vesikelinkorporation. Die mittlere Säule zeigt, dass die Pulsausübung im hypo-osmolaren Zustand der Zellsuspension fast eine Verdoppelung des Vesikeleinbaus bewirkt. Bei Pulsausübung im iso-osmolaren Zustand (rechte Säule) ergibt sich eine weniger starke Zunahme der Fluoreszenz. Dies wird damit erklärt, dass die Permeabilität der Membranoberfläche im iso-osmolaren Zustand geringer als im hypoosmolaren Zustand ist.

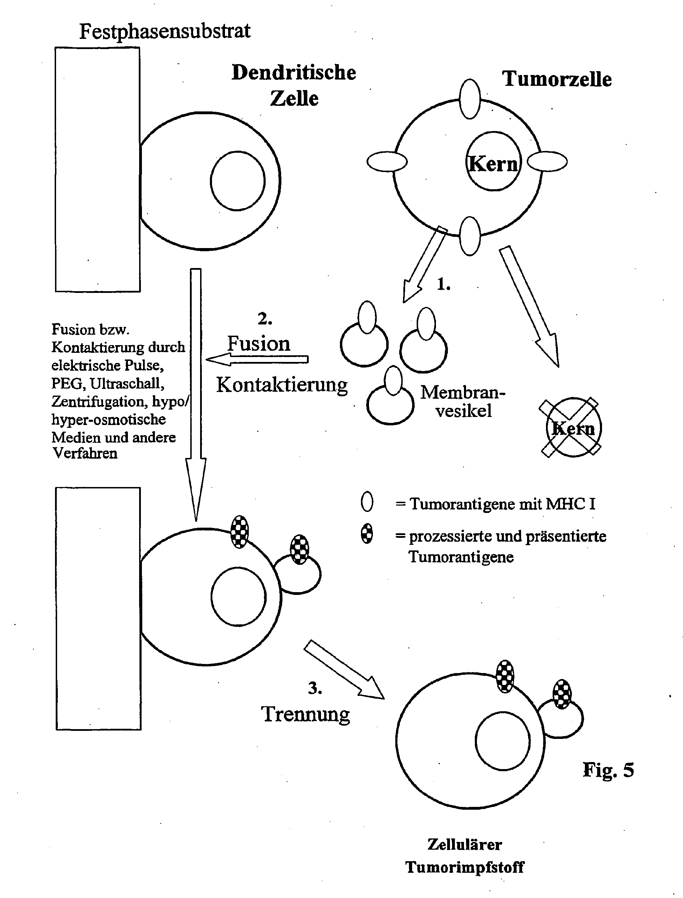

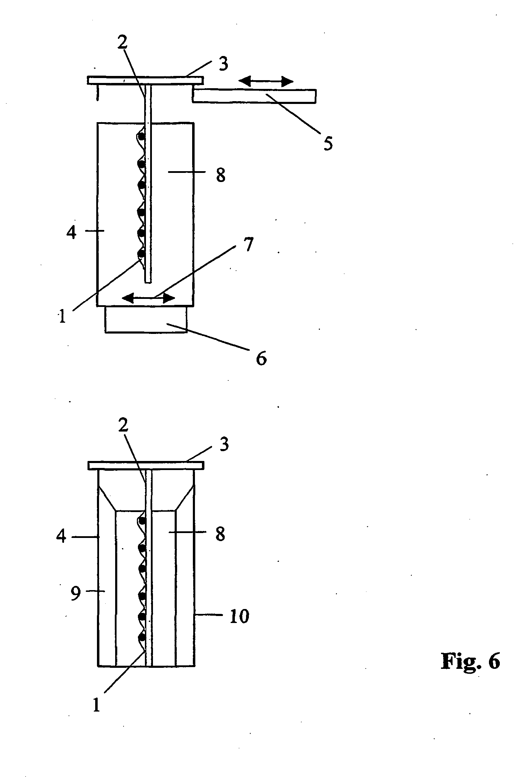

Die erfindungsgemäße Zellmodifizierung mit Festphasen-adsorbierten Zellen ist schematisch in den Figuren 5 und 6 illustriert. Zusätzlich zum Präparationsschritt 1. und Kontaktierungspunkt 2. ist ein Trennungsschritt 3. zur Abtrennung z. B. der modifizierten dendritischen Zellen vom Festphasensubstrat vorgesehen. Der Präparationsschritt 1. wird nach der oben beschriebenen Prozedur durchgeführt. Die Kontaktierungs- und Trennungsschritte 2. und 3. werden wie folgt realisiert.

### Kontaktierungsschritt 2./Trennungsschritt 3. (Festphasen-Modifizierung)

Zur Kontaktierung wird zunächst ein Festphasensubstrat mit adsorbierten dendritischen Zellen bereitgestellt. Das Festphasensubstrat wird dann mit der Vesikelsuspension in eine gemeinsame Suspension überführt, in der gemäß Figur 5 die Kontaktierung der Membranvesikeln mit den dendritischen Zellen unter Wirkung äußerer Kräfte erfolgt.

Die dem Körper des zu behandelnden Organismus entnommenen dendritischen Zellen werden als Monolage oder Submonolage auf dem Festphasensubstrat angeordnet. Dies erfolgt durch ein geeignetes Immobilisierungsverfahren. Die Immobilisierung umfasst beispielsweise ein mechanisches Aufbringen der Zellen auf die Festphase, gegebenenfalls unter Verwendung von Bindungsschichten auf der Festphasenoberfläche. Es können Zellen durch Unterdruck an eine Filtermembran angesaugt werden (analog zur Filtration). Für die Bindungsschichten werden beispielsweise Fibronection, Collagen, Poly-Lysin, Gelatine, Matrigel, FKS (Fötales Kälberserum) oder Alginat verwendet. Als Festphasensubstrat werden z. B. Kunststoff- oder Glassubstrate verwendet. Vorzugsweise werden die dendritischen Zellen auf mikroporösen Kunststoffmembranen immobilisiert. Als Membranen werden beispielsweise PET- oder PC-Membranen (Dicken rund 15 bis 25 µm, Porengrößen rund 0,4 bis 1 µm, Porosität rund 15 bis 20 %) verwendet. Bei diesen Substraten überdeckt jede Zelle im adhärenten Zustand vorzugsweise eine Pore, alternativ aber auch mehrere Poren.

Das Festphasensubstrat wird mit den Zellen in einer erfindungsgemäßen Vorrichtung, deren Einzelheiten unten unter Bezug auf Figur 6 erläutert werden, in einem Wechselwirkungsbereich gemeinsam mit der Suspension von erkrankten Zellen oder Zellbestandteilen, z. B. den oben erläuterten Membranvesikeln angeordnet. Im Wechselwirkungsbereich erfolgt die Übertragung der Antigene auf die dendritischen Zellen. Die Übertragung erfolgt durch osmotisch-induzierten oder elektrisch-induzierten Einbau der Vesikeln in die dendritischen Zellen analog zu den oben in Zusammenhang mit der Suspensions-Modifizierung beschriebenen Prozeduren.

Zur Ausübung äußerer Kräfte in Form von Strömungskräften wird die Membran mit den dendritischen Zellen und/oder die Suspensionsflüssigkeit im Wechselwirkungsbereich bewegt. Die Bewegung erfolgt vorzugsweise derart, dass im Wechselwirkungsbereich nahe der Oberfläche des Festphasensubstrats turbulente Strömungen auftreten, durch die in der Suspension befindliche Teilchen möglichst zahlreich und mit möglichst hohen Kräften gegen das Festphasensubstrat geströmt werden.

Beim elektrisch-induzierten Einbau werden z. B. 800 µl der Suspension mit den Vesikeln und dem eingetauchten Festphasensubstrat einem elektrischen Feldpuls ausgesetzt, der die äußeren Kräfte zur Modifizierung der dendritischen Zellen bildet. Die Ausübung des Feldpulses erfolgt auch hier vorzugsweise mit einer Elektrodeneinrichtung der erfindungsgemäßen Vorrichtung (siehe Figur 6). Alternativ zur Ausübung des Feldpulses können die Vesikeln auch dielektrophoretisch an die Festphasen-adsorbierten dentritischen Zellen herangeführt werden. Die genannten äußeren Kräfte werden durch die unter Wirkung von hochfrequenten elektrischen Feldern gebildeten Polarisationskräfte gebildet. Anschließend erfolgt eine Waschung mit PBS (280 mOsm), um die nicht-angekoppelten (noch freien) Membranvesikel zu entfernen.

Das nun vorliegende Festphasensubstrat trägt modifizierte dendritische Zellen, die beim Trennungsschritt 3. zur Bereitstellung des zellulären Tumorimpfstoffs vom Festphasensubstrat abgelöst werden (siehe Figur 5, rechts unten). Der Tumorimpfstoff enthält die Antigene der Tumorzelle direkt in der Membran oder in angehefteten Membranbestandteilen.

Die Trennung der modifizierten dendritischen Zellen vom Festphasensubstrat kann durch geeignete, an sich bekannte Ablösetechniken erfolgen. Es ist beispielsweise möglich, die Trennung durch Inkubation in einer hypoosmolaren Pufferlösung oder durch einen enzymatischen Abbau durchzuführen.

### Ausführungsformen einer Vorrichtung zur Festphasen-Modifizierung von Antigene präsentierenden Zellen

Figur 6 zeigt zwei Ausführungsformen von Vorrichtungen, die sich durch die Art der Kraftausübung bei der Wechselwirkung zwischen adsorbierten dendritischen Zellen und den suspendierten Teilchen unterscheiden. In beiden Fällen sind die dendritischen Zellen 1 auf dem Festphasensubstrat 2 angeordnet, das mit einem Rahmen (nicht dargestellt) an einem Träger 3 befestigt ist. Der Träger 3 ist mit einer Halterung so angeordnet, dass das Festphasensubstrat 2 in einen Flüssigkeits- oder Suspensionsbehälter (z. B. Küvette 4) hineinragt.

Zur Ausübung von Strömungskräften ist eine Schwenkeinrichtung 5, mit der der Träger 3 beweglich ist, und/oder eine Rühreinrichtung 6, 7 vorgesehen, mit der die Suspensionsflüssigkeit 8 im Innern der Küvette 4 beweglich ist. Die Rühreinrichtung 6, 7 wird beispielsweise durch einen magnetischen Rührer gebildet.

Zum elektrisch induzierten,Einbau der Vesikeln in die adsorbierten dendritischen Zellen ist gemäß dem unteren Teil von Figur 6 eine Elektrodeneinrichtung 9, 10 im Innern der Küvette 4 vorgesehen. Die Elektrodeneinrichtung besteht beispielsweise aus metallischen Beschichtungen (z. B. aus Platin), die auf der Innenseite der Küvette 4 angeordnet und elektrisch mit einer Steuereinrichtung (nicht dargestellt) verbunden sind. Das Festphasensubstrat 2 mit den Zellen 1 ragt in den Zwischenraum zwischen den Elektroden 9, 10, der mit der Suspensionsflüssigkeit 8 gefüllt ist. Der Träger 3 liegt auf dem oberen Rand der Küvette 4 auf.

Die Vorrichtung ist des weiteren mit Flüssigkeitszufuhreinrichtungen, Temperierungseinrichtungen und Manipulatoren ausgestattet, die anwendungsabhängig vorgesehen und in Figur 6 nicht dargestellt sind. Die Vorrichtung kann auch als Durchflusssystem gebildet sein, in dem laufend eine Modifizierung von Zellen gemäß dem erfindungsgemäßen Verfahren unter ständiger Zuführung von erkrankten Zellen oder Zellbestandteilen oder Antigene präsentierenden Zellen erfolgt.

Zur Zellmodifizierung in Suspensionen ist die Vorrichtung entsprechend ohne das Festphasensubstrat aufgebaut.

### Wichtige Merkmale der Erfindung sind im Folgenden zusammengefasst:

a) Die Erfinder haben festgestellt, dass eine passive Immunisierung oder Impfung sich überraschenderweise ohne Fusion von dendritischen Zellen mit erkrankten Zellen erreichen lässt. Es ist ausreichend, wenn eine Kontaktierung der Zellen bewirkt wird. Eine enge Kontaktierung zwischen Zellen beider Zelltypen lässt sich durch chemische Verbindungen oder durch physikalische Kräfte wie z. B. Dielektrophorese, Zentrifugation, Filtertechniken, usw., mit besonderem Vorteil erreichen, wenn die dendritischen Zellen sich in einem adsorbierten Zustand auf einem Festphasensubstrat befinden. Die Kontaktierung ist vorzugsweise so durchzuführen, dass bei Abschalten der mechanischen bzw. elektrischen Kräfte die kontaktierten Zellen sich nicht voneinander lösen.
b) Es reicht bereits aus, wenn die Membran der Tumorzellen in einen engen physikalischen oder chemischen Kontakt mit den dendritischen Zellen gebracht wird bzw. wenn bestrahlte (abgetötete) Tumorzellen verwendet werden.
c) Besonders vorteilhaft ist es, die Kontaktierung über Dielektrophorese in Mikrostrukturen durchzuführen. Dies besitzt insbesondere den Vorteil, dass in Mikroelektrodensystemen lokal hohe Felder erreicht werden.
d) Es ist vorteilhaft, eine Aufnahme von adherierenden Tumormembranstücken in die dendritischen Zellen vorzusehen und diese durch die Verwendung von stark hypo-osmolaren Lösungen zu erhöhen. Dies ergibt sich daraus, dass ggf. nach Übertragung in iso-osmolaren Lösungen eine Endozytose beobachtet wird. Die Verwendung iso-osmolarer Lösungen ist grundsätzlich jedoch auch möglich.
e) Besonders vorteilhaft ist es, eine feld-induzierte Endozytose auszulösen. Dies bedeutet, dass nach physikalischer oder chemischer Kontaktierung ein Durchbruchpuls in hypo-osmolarer Lösung induziert wird.

## Patentansprüche

1. Verfahren zur Modifizierung von Antigene präsentierenden Zellen, bei dem Antigene von erkrankten Zellen auf die Antigene präsentierenden Zellen übertragen werden, wobei die Antigene präsentierenden Zellen zu deren Modifizierung mit Antigen-tragenden Membrankomponenten von erkrankten Zellen durch Einführen in eine gemeinsame Suspension und Ausübung äußerer Kräfte in Kontakt gebracht werden,
wobei während der Präparation der Antigen-tragenden Membrankomponenten eine Abtrennung von Zellkernen und weiteren Zellbestandteilen erfolgte, so dass die Antigen-tragenden Membrankomponenten bei der Einführung in die Suspension frei von Zellkernen und weiteren Zellbestandteilen vorliegen,
wodurch gewährleistet wird, dass während des Kontaktes ausschließlich Membrankomponenten mit Antigenen ohne Zellkerne oder andere Bestandteile der erkrankten Zellen auf die Antigene präsentierenden Zellen übertragen werden, und wobei nach dem Kontakt mit erkrankten Zellen eine Abtrennung der modifizierten, Antigene präsentierenden Zellen von nicht übertragenen Membrankomponenten in der Suspension erfolgt.

2. Verfahren gemäß Anspruch 1, bei dem die Antigen-tragenden Membrankomponenten Membranvesikel darstellen, die aus Membranstücken der erkrankten Zellen gebildet sind und durch Homogenisierung und Zentrifugation der erkrankten Zellen erzeugt wurden, wobei die Homogenisierung und Zentrifugation unter solchen Bedingungen erfolgte, dass die Membranvesikel von den intakten Zellkernen getrennt wurden.

3. Verfahren gemäß Anspruch 1, bei dem die Antigene präsentierenden Zellen und die Antigen-tragenden Membrankomponenten der erkrankten Zellen in die gemeinsame Suspension frei suspendiert eingeführt werden.

4. Verfahren gemäß einem der vorhergehenden Ansprüche, bei dem die Antigene präsentierenden Zellen auf einem Festphasensubstrat angeordnet und die Antigen-tragenden Membrankomponenten frei suspendiert in die gemeinsame Suspension eingeführt werden, wobei nach der Übertragung der Membrankomponenten eine Abtrennung der modifizierten Zellen vom Festphasensubstrat erfolgt.

5. Verfahren gemäß einem der vorhergehenden Ansprüche, bei dem die Antigene präsentierenden Zellen mit den Antigen-tragenden Membrankomponenten durch Ausübung von Strömungskräften, z. B. Rühren oder Schütteln, dielektrophoretischen Kräften, Zentrifugationskräften, Filtertechniken, Sedimentation, hypo-osmolaren Schock und/oder optischen Kräften und chemischen Bindungen in Kontakt gebracht werden.

6. Verfahren gemäß Anspruch 1, bei dem die Antigene präsentierenden Zellen mit den Antigen-tragenden Membrankomponenten durch Einpipettieren in die Suspension und Erzeugung der Strömungskräfte durch Durchmischen in Kontakt gebracht werden.

7. Verfahren gemäß einen der vorhergehenden Ansprüche, bei dem die Suspension in einer isotonen Lösung gebildet wird.

8. Verfahren gemäß einem der Ansprüche 1 bis 6, bei dem die Suspension in einer hypotonen Lösung gebildet wird.

9. Verfahren gemäß einem der vorhergehenden Ansprüche, bei dem die Antigene präsentierenden Zellen und die Antigen-tragenden Membrankomponenten einer Feldbehandlung unterzogen werden.

10. Verfahren gemäß Anspruch 9, bei dem die Feldbehandlung die Ausübung mindestens eines elektrischen Hochspannungspulses und/oder die Ausübung von Polarisationskräften unter Wirkung von Dielektrophorese umfasst.

11. Verfahren gemäß einem der vorhergehenden Ansprüche, bei dem die Antigene präsentierenden Zellen dendritische Zellen, T-Zellen, B-Zellen oder Mastzellen umfassen.

12. Verfahren gemäß einem der vorhergehenden Ansprüche, bei dem die erkrankten Zellen Tumorzellen, Epithelzellen, Stammzellen, Knochenmarkzellen oder Virushüllen umfassen.

13. Verfahren gemäß Anspruch 12, bei dem eine Kurzzeitkontaktierung von dendritischen Zellen mit Antigen-tragenden Membrankomponenten der erkrankten Zellen erfolgt.

14. Verfahren gemäß Anspruch 1, bei dem mindestens eine Zellspezies mit äußeren Kräften zu den Antigen-tragenden Membrankomponenten bewegt wird, bis sie sich gegenseitig berühren.

15. Verfahren gemäß Anspruch 1, bei dem die äußeren Kräften so ausgeübt werden, dass die Zellen und die Antigen-tragenden Membrankomponenten aneinandergedrückt werden.

16. Verfahren gemäß einem der vorhergehenden Ansprüche, bei dem eine mit einem elektrischen Feld induzierte Endozytose erfolgt.

17. Zellulärer Tumorimpfstoff, der Antigene präsentierende Zellen enthält, die nach einem Verfahren gemäß einem der vorhergehenden Ansprüche modifiziert worden sind und Membrankomponenten mit Antigenen, jedoch keine Zellkerne oder andere Zellbestandteile der erkrankten Zellen, aufweisen.

18. Zellulärer Tumorimpfstoff nach Anspruch 17, der Antigene präsentierende Zellen enthält, in deren Zellmembranen Antigentragende Membrankomponenten von erkrankten Zellen, insbesondere Tumorzellen, eingebaut sind, die jedoch keine Zellkerne oder andere Zellbestandteile der erkrankten Zellen aufweisen.

## Claims

1. A method of modifying antigen-presenting cells, in which antigens of diseased cells are transferred to the antigen-presenting cells, wherein
the antigen-presenting cells for modifying the same are brought into contact with antigen-bearing membrane components of diseased cells by introduction into a shared suspension and the exertion of external forces, wherein during the preparation of the antigen-bearing membrane components a separation of cell nuclei and further cell components has been performed such that the antigen-bearing components are free of cell nuclei and further cell components when introduced into said suspension, which ensures that during the contact exclusively membrane components with antigens free of cell nuclei or other components of the diseased cells are transferred onto the antigen-presenting cells, and wherein after the contact with diseased cells a separation of the modified antigen-presenting cells from membrane components in the suspension which have not been transferred is performed.

2. The method according to claim 1, wherein the antigen-bearing membrane components represent membrane vesicles which are formed from membrane parts of the diseased cells and which have been produced through homogenization and centrifugation of the diseased cells, wherein said homogenization and centrifugation have been performed under such conditions that the membrane vesicles were separated from the intact cell nuclei.

3. The method according to claim 1, wherein the antigen-presenting cells and the antigen-bearing membrane components of the diseased cells are incorporated into the shared suspension while freely suspended.

4. The method according to any one of the preceding claims, wherein the antigen-presenting cells are positioned on a solid-phase substrate and the antigen-bearing membrane components are incorporated into the shared suspension while freely suspended, detachment of the modified cells from the solid-phase substrate being performed after the transfer of the membrane components.

5. The method according to any one of the preceding claims, wherein the antigen-presenting cells are brought into contact with the antigen-bearing membrane components by exerting flow forces, for example, stirring or shaking, dielectrophoretic forces, centrifugation forces, filter techniques, sedimentation, hypoosmolar shock, and/or optical forces and chemical bonds.

6. The method according to Claim 1, wherein the antigen-presenting cells are brought into contact with the the antigen-bearing membrane components through pipetting into the suspension and production of the flow forces through mixing.

7. The method according to any one of the preceding claims, wherein the suspension is produced in an isotonic solution.

8. The method according to any one of Claims 1 to 6, wherein the suspension is produced in a hypotonic solution.

9. The method according to any one of the preceding claims, wherein the antigen-presenting cells and the antigen-bearing membrane components are subjected to a field treatment.

10. The method according to claim 9, wherein the field treatment includes the application of at least one electrical high-voltage pulse and/or the application of polarization forces under the effect of dielectrophoresis.

11. The method according to any one of the preceding claims, wherein the antigen-presenting cells include dendritic cells, T-cells, B-cells, or mast cells.

12. The method according to any one of the preceding claims, wherein the diseased cells include tumor cells, epithelial cells, stem cells, bone marrow cells, or virus envelopes.

13. The method according to claim 12, wherein there is short-term contact of the dendritic cells with the antigen-bearing membrane components of the diseased cells.

14. The method according to Claim 1, wherein at least one cell species is moved using external forces toward the the antigen-bearing membrane components until they come into mutual contact.

15. The method according to Claim 1, wherein the external forces are exerted in such a way that the cells and the antigen-bearing membrane components are pressed against one another.

16. The method according to any one of the preceding claims, wherein an endocytosis, induced by an electric field, is performed.

17. A cellular tumor vaccine, which contains antigen-presenting cells, which were modified according to a method according to any one of the preceding claims and which contain membrane components with antigens but no cell nuclei or other cellular components of the diseased cells.

18. The cellular tumor vaccine according to claim 17, which contains antigen-presenting cells, into whose cell membranes antigen-bearing membrane components of diseased cells, particularly tumor cells, are incorporated, but which do not contain cell nuclei or other cellular components of the diseased cells.

## Revendications

1. Procédé de modification de cellules présentatrices d'antigènes, où des antigènes de cellules malades sont transférés aux cellules présentatrices d'antigènes, les cellules présentatrices d'antigènes étant en vue de leur modification mises en contact dans une suspension commune et sous l'action de forces externes avec des composants de membrane porteurs d'antigènes de cellules malades, une séparation de noyaux cellulaires et d'autres composants cellulaires ayant eu lieu pendant la préparation des composants de membrane porteurs d'antigènes, de telle manière que les composants de membrane porteurs d'antigènes sont exempts de noyaux cellulaires et d'autres composants cellulaires lors de leur ajout dans la suspension, ce qui assure que seuls des composants de membrane avec des antigènes sans noyaux cellulaires ou sans autres composants des cellules malades sont transférés aux cellules présentatrices d'antigènes pendant le contact, et une séparation étant réalisée dans la suspension, après le contact avec des cellules malades, entre les cellules présentatrices d'antigènes modifiées et les composants de membrane non transférés.

2. Procédé selon la revendication 1, où les composants de membrane porteurs d'antigènes représentent des vésicules de membrane ayant été formées à partir de fragments de membrane des cellules malades et générées par homogénéisation et centrifugation dans des conditions telles que les vésicules de membrane ont été séparées des noyaux cellulaires intacts.

3. Procédé selon la revendication 1, où les cellules présentatrices d'antigènes et les composants de membrane porteurs d'antigènes des cellules malades sont librement suspendus dans la suspension commune.

4. Procédé selon l'une des revendications précédentes, où les cellules présentatrices d'antigènes sont disposées sur un substrat de phase solide et où les composants de membrane porteurs d'antigènes sont librement suspendus dans la suspension commune, une séparation étant réalisée après transfert des composants de membrane entre les cellules modifiées et le substrat de phase solide.

5. Procédé selon l'une des revendications précédentes, où les cellules présentatrices d'antigènes sont mises en contact avec les composants de membrane porteurs d'antigènes sous l'action de forces d'écoulement, notamment par agitation ou secouage, forces diélectrophorétiques, forces centrifuges, techniques de filtration, sédimentation, choc hypoosmolaire et/ou forces optiques et liaisons chimiques.

6. Procédé selon la revendication 1, où les cellules présentatrices d'antigènes sont mises en contact avec les composants de membrane porteurs d'antigènes par pipetage dans la suspension et génération par mélange des forces d'écoulement.

7. Procédé selon l'une des revendications précédentes, où la suspension est formée dans une solution isotone.

8. Procédé selon l'une des revendications 1 à 6, où la suspension est formée dans une solution hypotone.

9. Procédé selon l'une des revendications précédentes, où les cellules présentatrices d'antigènes et les composants de membrane porteurs d'antigènes sont soumis à un traitement de champ.

10. Procédé selon la revendication 9, où le traitement de champ comprend l'application d'au moins une impulsion électrique à haute tension et/ou l'application de forces de polarisation par diélectrophorèse.

11. Procédé selon l'une des revendications précédentes, où les cellules présentatrices d'antigènes comprennent des cellules dendritiques, des cellules T, des cellules B ou des mastocytes.

12. Procédé selon l'une des revendications précédentes, où les cellules malades comprennent des cellules tumorales, des cellules épithéliales, des cellules souches, des cellules médullaires ou des capsides virales.

13. Procédé selon la revendication 12, où est réalisée une mise en contact de courte durée de cellules dendritiques avec les composants de membrane porteurs d'antigènes des cellules malades.

14. Procédé selon la revendication 1, où au moins une espèce cellulaire est mue par des forces externes vers les composants de membrane porteurs d'antigènes, jusqu'à obtenir un contact réciproque.

15. Procédé selon la revendication 1, où les forces externes sont exercées de manière à serrer les unes contre les autres les cellules et les composants de membrane porteurs d'antigènes.

16. Procédé selon l'une des revendications précédentes, où une endocytose est réalisée, induite par un champ électrique.

17. Vaccin cellulaire anti-tumoral contenant des cellules présentatrices d'antigènes qui ont été modifiées par un procédé selon l'une des revendications précédentes et qui présentent des composants de membrane porteurs d'antigènes mais exempts de noyaux cellulaires ou d'autres composants cellulaires des cellules malades.

18. Vaccin cellulaire anti-tumoral selon la revendication 17, contenant des cellules présentatrices d'antigènes dans les membranes cellulaires desquelles sont intégrés des composants de membrane porteurs d'antigènes de cellules malades, en particulier de cellules tumorales, lesquels sont toutefois exempts de noyaux cellulaires ou d'autres composants cellulaires des cellules malades.
